# EUROPÄISCHE PATENTSCHRIFT

(11) **EP 0 899 270 B1**
(45) Veröffentlichungstag und Bekanntmachung des Hinweises auf die Patenterteilung: **21.05.2008**
(21) Anmeldenummer: 98115652.4
(22) Anmeldetag: 20.08.1998
(51) Int. Cl.: C07D 403/06, A61K 31/495

(54) **Indolmethyl-N,N'-bisacylpiperazine als Neurokininrezeptorantagonisten**
Indolylmethyl-N,N'-bisacylpiperazines as neurokinine receptor antagonists
Indolylmethyl-N,N'-bisacylpipérazines comme antagonistes des récepteurs de neurokinine

(30) Priorität: 27.08.1997 DE 19737274; 04.06.1998 DE 19824865
(43) Veröffentlichungstag der Anmeldung: 03.03.1999
(73) Patentinhaber: Solvay Pharmaceuticals GmbH, 30173 Hannover (DE)
(72) Erfinder: Jasserand, Daniel, 30625 Hannover (DE); David, Samuel, 30559 Hannover (DE); Antel, Jochen, 31848 Bad Münder (DE); Brückner, Reinhard, 30559 Hannover (DE); Eeckhout, Christian, 29690 Lindwedel (DE); Bielenberg, Gerhard-Wilhelm, 31061 Alfeld (DE)
(74) Vertreter: Gosmann, Martin

(56) Entgegenhaltungen:
- EP-A- 0 655 442
- EP-A- 0 832 887
- WO-A-96/37489
- WO-A-97/11069
- WO-A-97/28141

## Beschreibung

Die Erfindung betrifft neue Harnstoffderivate, in welchen ein Stickstoffatom Bestandteil eines Piperazinringes ist und das andere durch einen Benzylaminoethylrest substituiert ist. Diese neuen Harnstoffderivate zeichnen sich durch Neurokinin-Rezeptor-antagonistische Eigenschaften mit einem zur Behandlung von funktionellen und entzündlichen Störungen des gastrointestinalen Traktes von größeren Säugetieren, insbesondere Menschen, günstigen Wirkungsprofil aus. Ferner betrifft die Erfindung diese neuen Verbindungen enthaltende Arzneimittel sowie Verfahren zur Herstellung dieser Verbindungen.

Der Erfindung liegt die Aufgabe zugrunde, neue Wirkstoffe zur Behandlung von funktionellen und entzündlichen Störungen im gastrointestinalen Trakt zu entwickeln.

Aus der Europäischen Patentanmeldung, Veröffentlichungs-Nr. 655 442, sind bereits Piperazinderivate mit Neurokinin-Rezeptor-antagonistischen Eigenschaften bekannt.

Es wurde nun gefunden, daß eine Gruppe von neuen Piperazinderivaten, welche in 2-Stellung durch einen Indolylmethylrest substituiert sind, und in welchen der Stickstoff in 4-Stellung des Piperazinringes Teil eines einen Benzylaminoethylsubstituenten tragenden Harnstoffgerüstes ist, ein Wirkungsprofil zeigen, welches sie zur Behandlung von funktionellen und entzündlichen Störungen des gastrointestinalen Traktes geeignet macht. Die erfindungsgemäße Gruppe von Substanzen zeichnet sich ferner durch eine gute Verträglichkeit und eine gute orale Bioverfügbarkeit aus.

Gegenstand der Erfindung sind daher neue Verbindungen der Formel I, 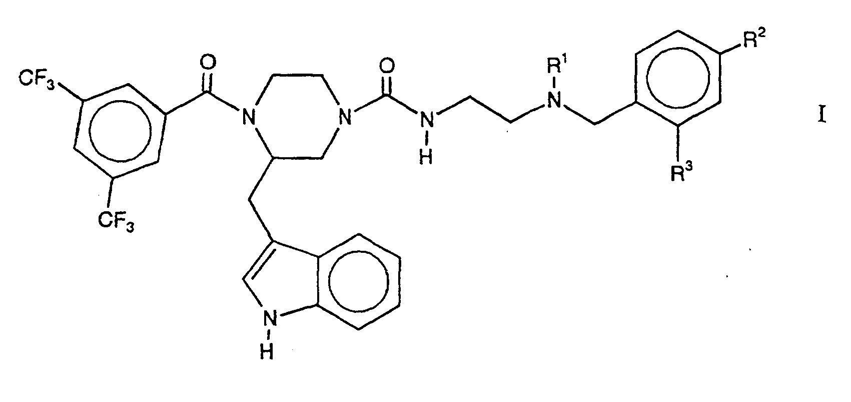 worin
- R¹: Wasserstoff oder niederes Alkyl bedeutet,
- R²: Wasserstoff oder Halogen bedeutet und
- R³: Wasserstoff oder niederes Alkoxy bedeutet
und deren physiologisch verträgliche Säureadditionssalze sowie aus diesen Verbindungen herstellbare Arzneimittel.

Sofern in Verbindungen der Formel I die Substituenten niederes Alkyl bedeuten oder enthalten, kann dieses geradkettig oder verzweigt sein und 1 bis 4, vorzugsweise 1 bis 2, Kohlenstoffatome enthalten.

Sofern R¹ für niederes Alkyl steht, ist Methyl bevorzugt.

Sofern der Substituent R² Halogen bedeutet, ist Fluor bevorzugt.

Sofern der Substituent R³ niederes Alkoxy bedeutet, ist Methoxy bevorzugt.

Bevorzugt sind Verbindungen der Formel I, worin R² für Wasserstoff steht und R³ Methoxy bedeutet oder Verbindungen der Formel I, worin R² Fluor bedeutet und R³ für Wasserstoff steht.

Der 1H-Indol-3-yl-methylrest ist bevorzugt in 2R-Stellung des Piperazinringes angeordnet.

Die Verbindungen können auf an sich bekannte Weise hergestellt werden. Besonders günstig können die Verbindungen der Formel I hergestellt werden, indem man
a) die Verbindung der Formel II, 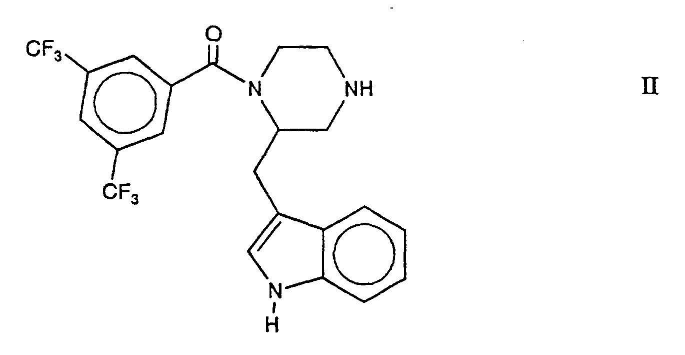 mit einer reaktiven Carbonylverbindung der allgemeinen Formel III, 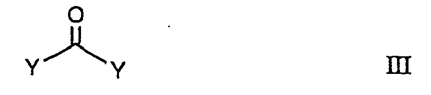 worin Y eine durch nucleophilen Angriff eines primären oder sekundären Amins verdrängbare Fluchtgruppe bedeutet, zu einer Carbamoyl-Verbindung der allgemeinen Formel IV, 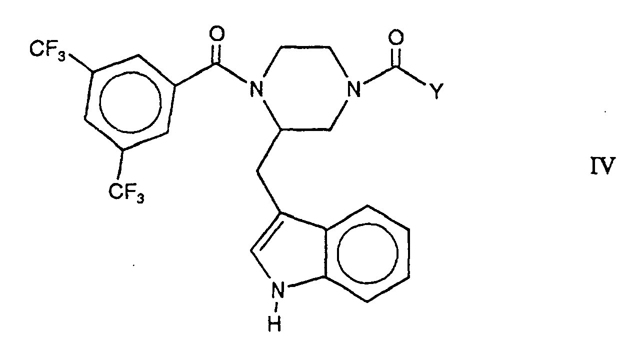 worin Y die obige Bedeutung besitzt, umsetzt, sofern durch Abspaltung der Fluchtgruppe Y aus einer erhaltenen Verbindung der Formel IV eine Säure entstehen kann, zu der Verbindung der Formel IV eine nicht-nucleophile organische Base zugibt, und die Verbindung der Formel IV anschließend mit einer Verbindung der allgemeinen Formel V, 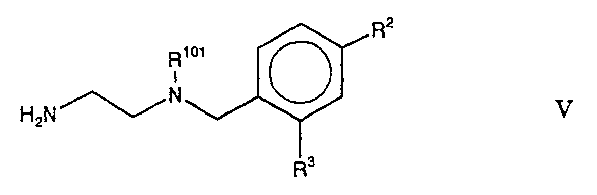 worin R¹⁰¹ niederes Alkyl oder eine Aminoschutzgruppe bedeutet und R² und R³ obige Bedeutungen besitzen, umsetzt und eine allfällige Schutzgruppe R¹⁰¹ anschließend wieder abspaltet oder
b) die Verbindung der Formel II mit einer Verbindung der allgemeinen Formel VI, 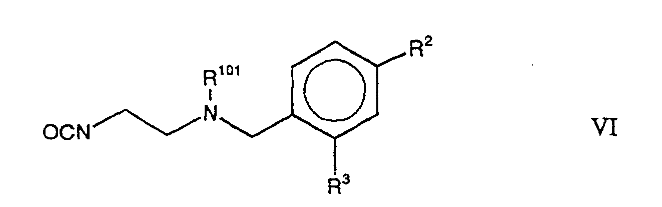 worin R¹⁰¹, R² und R³ obige Bedeutungen besitzen, umsetzt und eine allfällige Aminoschutzgruppe R¹⁰¹ nachfolgend wieder abspaltet
und gewünschtenfalls erhaltene Verbindungen der Formel I, worin R¹ Wasserstoff bedeutet, zu Verbindungen der Formel I, worin R¹ niederes Alkyl bedeutet, alkyliert und erhaltene Verbindungen der Formel I gewünschtenfalls in ihre Säureadditionssalze überführt oder Säureadditionssalze in freie Verbindungen der Formel I überführt.

Zweckmäßigerweise kann gemäß Verfahrensvariante a) zuerst eine Verbindung der Formel II mit einer reaktiven Carbonylverbindung der Formel III zu einer Carbamoyl-Verbindung der Formel IV umgesetzt werden, welche direkt in situ, gegebenenfalls nach Zugabe einer nicht-nucleophilen organischen Base, mit einem Amin der Formel V umgesetzt werden kann. Als Fluchtgruppen Y in Verbindungen der Formel III eignen sich beispielsweise Halogene, vorzugsweise Chlor, Trihalomethoxygruppen, vorzugsweise Trichlormethoxygruppen, oder auch Imidazolylgruppen. Vorzugsweise können als reaktive Carbonylverbindungen der Formel III Phosgen, Bis-(trichlormethyl)-carbonat (Triphosgen), Chlorameisensäure-trichlormethylester (Diphosgen) oder Carbonyldiimidazol eingesetzt werden. Bei der Umsetzung eines Amins der Formel V mit einer Carbamoyl-Verbindung der Formel IV wird die Fluchtgruppe Y aus der Verbindung der Formel IV verdrängt. Sofern aus der hierbei freigesetzten Gruppe Y eine Säure entstehen kann, kann der Verbindung der Formel IV vor der Umsetzung mit der Verbindung der Formel V zweckmäßig eine nicht-nucleophile organische Base zugesetzt werden. Sofern Y beispielsweise für Chlor steht, kann die bei der Abspaltung von Y entstehende Chlorwasserstoffsäure durch Zugabe einer vorgenannten Base abgefangen werden. Als nicht-nucleophile Basen eignen sich in dem Reaktionsgemisch lösliche organische Basen wie tertiäre Stickstoffbasen, beispielsweise stickstoffhaltige N-alkylierte Heterocyclen wie N-Niederalkyl-Morpholin oder N-Niederalkyl-Piperidin oder tertiäre Niederalkylamine und Pyridine, wie z. B. Triethylamin, Tripropylamin, Diisopropylethylamin, Pyridin, 4-Dimethylaminopyridin, 4-Diethylaminopyridin oder 4-Pyrrolidinopyridin. Im Überschuß eingesetzte Basen können auch als Lösungsmittel verwendet werden. Die Reaktionsfolge kann als Eintopfreaktion in einem polaren aprotischen Lösungsmittel wie einem teilhalogenierten niederen Kohlenwasserstoff, beispielsweise Dichlormethan, bei Temperaturen zwischen -20 °C und Raumtemperatur, bevorzugt bei Raumtemperatur durchgeführt werden.

Die Umsetzung der Verbindung der Formel II mit einem Isocyanat der Formel VI kann gemäß Verfahrensvariante b) auf an sich bekannte Weise erfolgen. Die Verbindungen der Formel VI können beispielsweise aus den Aminen der Formel V durch Umsetzung mit geeigneten reaktiven Carbonylverbindungen erhalten werden. Als reaktive Carbonylverbindungen eignen sich beispielsweise die Verbindungen der Formel III. Zweckmäßig wird aus einem Amin der Formel V zuerst ein Isocyanat der Formel VI hergestellt und dieses wird dann direkt in situ mit einer Verbindung der Formel II umgesetzt. Die Reaktionsfolge kann unter den oben zur Herstellung von Verbindungen der Formel I gemäß Verfahrensvariante a) angegebenen Bedingungen als Eintopf-Reaktion ausgeführt werden. Zweckmäßig kann dem Reaktionsgemisch ein säurebindendes Reagens zugesetzt werden. Als säurebindende Reagenzien eignen sich die oben angegebenen nicht-nucleophilen Basen.

Als Aminoschutzgruppen R¹⁰¹ kommen an sich, beispielsweise aus der Peptidchemie bekannte Aminoschutzgruppen in Frage, welche nach an sich bekannten Methoden eingeführt und wieder abgespalten werden können. Geeignete Schutzgruppen sind beispielsweise bekannt aus J.A.W. McOmie "Protective Groups in Organic Chemistry" Plenum Press 1973 oder T.W. Green und P.G.M. Wuts "Protective Groups in Organic Synthesis", Wiley and Sons 1991.

Beispielsweise eignen sich als Aminoschutzgruppen R¹⁰¹ im sauren und im alkalischen Milieu weitgehend stabile Gruppen, welche unter hydrogenolytischen Bedingungen abgespalten werden können. Hierzu zählen beispielsweise Phenylniederalkyloxycarbonylgruppen wie die Benzyloxycarbonylgruppe (im folgenden als CbO abgekürzt). Bevorzugt kann als Aminoschutzgruppe R¹⁰¹ die Benzyloxycarbonylgruppe verwendet werden, welche auf an sich bekannte Weise, z. B. durch katalytische Hydrierung, abgespalten werden kann, um Verbindungen der Formel I zu erhalten, worin R¹ Wasserstoff bedeutet. Die Abspaltung der Schutzgruppe kann in einem unter den Reaktionsbedingungen inerten organischen Lösungsmittel wie einem niederen aliphatischen Ether, beispielsweise Tetrahydrofuran (im folgenden als THF abgekürzt) oder Diethylether, niederen Alkanolen, beispielsweise Methanol oder Ethanol, oder organischen Säuren, beispielsweise niederen aliphatischen Carbonsäuren wie Essigsäure, oder in Gemischen dieser Lösungsmittel und in Anwesenheit eines Hydrierungskatalysators erfolgen. Als Hydrierungskatalysatoren eignen sich beispielsweise Edelmetallkatalysatoren wie Palladium auf Aktivkohle. Zweckmäßig wird die Reaktion bei Raumtemperatur ausgeführt. Ein zur Hydrierung geeigneter Wasserstoffdruck beträgt zwischen 2 und 7 bar, bevorzugt zwischen 3 und 5 bar.

Die Verbindungen der Formel I, worin R¹ Wasserstoff bedeutet, können gewünschtenfalls nach an sich zur Aminoalkylierung bekannten Methoden in Verbindungen der Formel I überführt werden, worin R¹ niederes Alkyl bedeutet. Hierfür können die Verbindungen der Formel I beispielsweise durch Umsetzung mit niederen aliphatischen Aldehyden wie Formaldehyd reduktiv alkyliert werden. Die Umsetzung kann unter an sich zur reduktiven Alkylierung von Aminen üblichen Bedingungen, beispielsweise unter den Bedingungen einer katalytischen Hydrierung, durchgeführt werden. Als Hydrierungskatalysatoren eignen sich Metallkatalysatoren wie Raney-Nickel. Als Lösungsmittel können vorzugsweise niedere Alkanole verwendet werden. Die katalytische Hydrierung kann unter den vorstehend für die hydrogenolytische Abspaltung von Aminoschutzgruppen R¹⁰¹ beschriebenen Bedingungen durchgeführt werden.

Eine andere Möglichkeit der Alkylierung ist die Umsetzung von Verbindungen der Formel I, worin R¹ Wasserstoff bedeutet, mit niederen aliphatischen Alkylhalogeniden wie Alkylbromiden oder Alkyljodiden, vorzugsweise Methyljodid, Alkylsulfaten oder Alkylsulfonsäureestern, unter für nucleophile Substitutionsreaktionen allgemein üblichen Bedingungen. Die Reaktion kann in einem polaren aprotischen Lösungsmittel wie Dimethylformamid (im folgenden als DMF abgekürzt), Dimethylsulfoxid (im folgenden als DMSO abgekürzt) oder Acetonitril bei Temperaturen zwischen -20 °C und 100 °C, bevorzugt zwischen 60 °C und 90 °C und unter Verwendung eines säurebindenden Reagenzes durchgeführt werden. Als säurebindende Reagenzien eignen sich beispielsweise die oben bei der Umsetzung der Verbindungen der Formel IV mit den Verbindungen der Formel V angegebenen organischen Basen.

Als physiologisch verträgliche Salze von Verbindungen der Formel I kommen deren Salze mit anorganischen Säuren, beispielsweise Schwefelsäure, Phosphorsäuren oder Halogenwasserstoffsäuren, vorzugsweise Chlorwasserstoffsäure, oder mit organischen Säuren, beispielsweise niederen aliphatischen Mono-, Di- oder Tricarbonsäuren wie Maleinsäure, Fumarsäure, Milchsäure, Weinsäure, Zitronensäure, oder mit Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure, in Frage.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in pharmakologisch verträgliche Säureadditionssalze überführt werden.

Die Verbindungen der Formel I enthalten ein chirales Kohlenstoffatom, nämlich das den 1H-Indol-3-yl-methylrest tragende Kohlenstoffatom in 2-Stellung des Piperazin-Grundgerüstes. Die Verbindungen der Formel I können somit in mehreren stereoisomeren Formen vorliegen. Die vorliegende Erfindung umfaßt sowohl die Gemische optischer Isomere als auch die isomerenreinen Verbindungen der Formel I. Bevorzugt sind Verbindungen der Formel I, worin der Indolylmethylrest in 2R-Stellung des Piperazinringes angeordnet ist.

Falls bei der Synthese der Verbindungen der Formel I Gemische optischer Isomere der Ausgangsverbindung der Formel II eingesetzt werden, werden auch die Verbindungen der Formel I in Form von Gemischen optischer Isomere erhalten. Ausgehend von stereochemisch einheitlichen Formen der Ausgangsverbindung können auch stereochemisch einheitliche Verbindungen der Formel I erhalten werden. Die stereochemisch einheitlichen Verbindungen der Formel I können aus den Gemischen optischer Isomere in an sich bekannter Weise erhalten werden, z. B. durch chromatographische Trennung an chiralen Trennmaterialien oder durch Umsetzung mit geeigneten optisch aktiven Säuren, beispielsweise Weinsäure oder Campher-10-sulfonsäure, und anschließender Auftrennung in die optisch aktiven Antipoden durch fraktionierte Kristallisation der gewonnenen diastereomeren Salze.

Die beiden möglichen Enantiomere der Verbindung der Formel II sind aus der EP-A 655 422 bekannt und können nach den in dieser Patentanmeldung beschriebenen Verfahren oder analog zu diesen Verfahren hergestellt werden.

Die Amine der Formel V können aus den zweifach aminogeschützten Diaminoverbindungen der allgemeinen Formel VII, 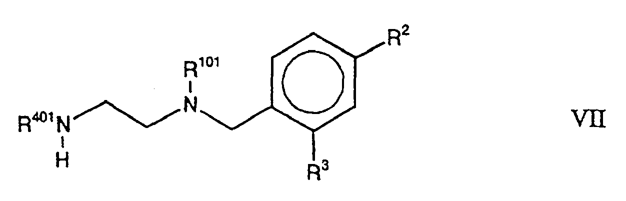 worin R¹⁰¹, R² und R³ obige Bedeutungen besitzen und R⁴⁰¹ für eine Aminoschutzgruppe steht, erhalten werden, indem man aus Verbindungen der Formel VII in an sich bekannter Weise die Aminoschutzgruppe R⁴⁰¹ selektiv abspaltet.

Als Aminoschutzgruppen R⁴⁰¹ eignen sich allgemein, beispielsweise aus der Peptidchemie, bekannte Aminoschutzgruppen wie sie aus den vorstehend angegebenen Quellen bekannt sind. Beispielsweise eignen sich als Aminoschutzgruppen R⁴⁰¹ im mindestens mäßig sauren Milieu, beispielsweise durch Zugabe von p-Toluolsulfonsäure, Trifluoressigsäure oder gasförmiger oder in Lösungsmitteln gelöster Salzsäure, selektiv abspaltbare Gruppen, welche gegen hydrogenolytische und alkalische Bedingungen weitgehend stabil sind. Hierzu zählen beispielsweise verzweigte Niederalkyloxycarbonylgruppen wie die tert.-Butyloxycarbonylgruppe (im folgenden als BOC abgekürzt). Vorzugsweise kann R⁴⁰¹ für die tert.-Butyloxycarbonylgruppe stehen.

Verbindungen der Formel VII können auf an sich bekannte Weise, beispielsweise durch Reduktion von Amiden der allgemeinen Formel VIII, 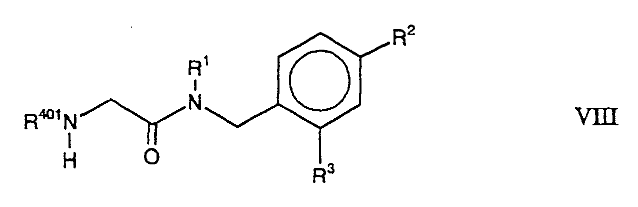 worin R¹, R², R³ und R⁴⁰¹ obige Bedeutungen besitzen, und, sofern R¹ für Wasserstoff steht, nachfolgende Einführung einer Schutzgruppe R¹⁰¹, erhalten werden. Die Reduktion kann mit komplexen Alkalimetallhydriden wie Lithiumaluminiumhydrid als Reduktionsmittel durchgeführt werden. Als Lösungsmittel eignen sich unter den Reaktionsbedingungen inerte organische Lösungsmittel wie niedere aliphatische Ether, beispielsweise Dioxan, THF oder Diethylether oder Gemische dieser Lösungsmittel. Ein geeigneter Temperaturbereich liegt zwischen -20 °C und der Siedetemperatur des Reaktionsgemisches. Vorzugsweise kann die Reduktion bei Raumtemperatur durchgeführt werden.

Die Amide der Formel VIII können durch Umsetzung von aminogeschützten ω-Aminocarbonsäuren der allgemeinen Formel IX, 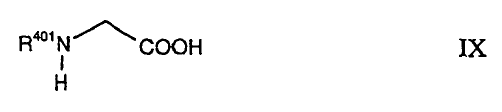 worin R⁴⁰¹ obige Bedeutung besitzt, mit den Aminen der allgemeinen Formel X, 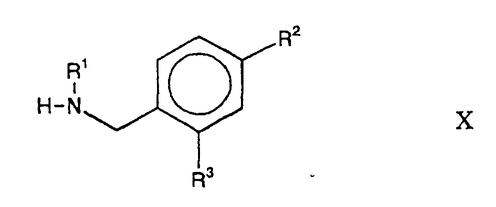 worin R¹, R² und R³ obige Bedeutungen besitzen, unter zur Bildung von Amidgruppierungen durch Aminoacylierung üblichen Methoden hergestellt werden. Als Acylierungsmittel können die Säuren der Formel IX oder deren reaktionsfähige Derivate eingesetzt werden. Als reaktionsfähige Derivate kommen insbesondere gemischte Säureanhydride und Säurechloride oder Säurebromide der Säuren der Formel IX oder gemischte Ester der Säuren der Formel IX mit Chlorameisensäure oder mit organischen Sulfonsäuren, beispielsweise aromatischen Sulfonsäuren wie durch niederes Alkyl oder Halogen substituierten Benzolsulfonsäuren, z.B. p-Toluolsulfonsäure, in Frage. Die Acylierung kann in einem unter den Reaktionsbedingungen inerten organischen Lösungsmittel bei Temperaturen zwischen -20 °C und Raumtemperatur, vorzugsweise bei Raumtemperatur erfolgen. Als Lösungsmittel eignen sich aromatische Kohlenwasserstoffe wie Benzol oder Toluol, aliphatische Ether wie Diethylether, THF oder Dioxan, teilhalogenierte niedere Kohlenwasserstoffe wie Dichlormethan oder Gemische dieser Lösungsmittel.

Die Acylierung kann zweckmäßig, insbesondere wenn als Acylierungsmittel ein Säurehalogenid der Säuren der Formel IX verwendet wird, in Gegenwart eines säurebindenden Reagenzes durchgeführt werden. Als säurebindende Reagenzien eignen sich die oben bei der Umsetzung der Carbamoyl-Verbindungen der Formel IV mit den Verbindungen der Formel V angegebenen nicht-nucleophilen organischen Basen.

Falls als Acylierungsmittel die Säuren der Formel IX selbst eingesetzt werden, kann die Umsetzung der Amine der Formel X mit den Säuren der Formel IX zweckmäßig auch in Gegenwart eines aus der Peptidchemie als zur Amidbildung geeignet bekannten Kopplungsreagenzes durchgeführt werden. Als Beispiel von Kopplungsreagenzien, welche die Amidbildung mit den freien Säuren dadurch fördern, daß sie mit der Säure in situ unter Bildung eines reaktionsfähigen Säurederivates reagieren, seien insbesondere genannt: Alkylcarbodiimide, z. B. Cycloalkylcarbodiimide wie Dicyclohexylcarbodiimid oder 1-Ethyl-3-[(dimethylamino)-propyl]-carbodiimid, Diisopropylcarbodiimid und Carbonyldiimidazol. Die Umsetzung in Gegenwart eines Kopplungsreagenzes kann zweckmäßig bei Temperaturen zwischen -30 °C und +50 °C in Lösungsmitteln wie halogenierten Kohlenwasserstoffen und/oder aromatischen Lösungsmitteln wie gegebenenfalls substituierten Benzolen, und gegebenenfalls in Gegenwart einer säurebindenden organischen Verbindung, beispielsweise einer vorstehend beschriebenen nicht-nucleophilen Stickstoffbase durchgeführt werden. Die Säuren der Formel IX stellen aminogeschützte Derivate der 2-Aminoessigsäurederivate dar, welche in ungeschützter Form bekannt ist und welche nach an sich bekannten Methoden in die aminogeschützten Derivate überführt werden kann.

Die Verbindungen der Formel X sind bekannt oder können auf an sich bekannte Weise aus bekannten Verbindungen hergestellt werden.

Die Verbindungen der Formel I und ihre Säureadditionssalze besitzen Neurokinin(= NK)-Rezeptor-antagonistische Eigenschaften und sind zur Behandlung von Krankheitszuständen geeignet, bei denen Neurokinine als Überträgerstoffe beteiligt sind. Dabei zeichnet sich die erfindungsgemäße Gruppe von Verbindungen durch ein besonders günstiges selektives Wirkprofil aus, welches gekennzeichnet ist durch eine hohe Affinität zu NK-1-Rezeptoren mit einer im Verhältnis hierzu geringeren Affinität zu NK-2-Rezeptoren. Ferner weisen die Verbindungen eine gute orale Wirksamkeit auf.

Aufgrund ihres Wirkprofils eignet sich die erfindungsgemäße Gruppe von Substanzen insbesondere zur Hemmung von Vorgängen, an denen an NK-1-Rezeptoren bindende Neurokinine wie Substanz P beteiligt sind. Somit eignen sich die Substanzen selektiv zur Behandlung von Krankheitszuständen, bei welchen Substanz P beteiligt ist. Substanz P spielt beispielsweise eine Rolle bei der Schmerzübertragung, Emese, neurogenen Engtzündungen, Harnblasenentzündungen, entzündlichen Gelenkserkrankungen und asthmatischen Beschwerden. Wegen der in vorteilhafter Weise auf den gastrointestinalen Trakt gerichteten Wirkung eignet sich das Wirkprofil der Substanzen für die Behandlung von funktionellen und entzündlichen Störungen im gastrointestinalen Trakt. Weiterhin wird von Verbindungen, welche neben einer hohen Affinität zu NK-1-Rezeptoren auch eine gewisse Affinität zu NK-2-Rezeptoren aufweisen, allgemein angenommen, daß diese beiden Wirkungskomponenten einen günstigen synergistischen Einfluß auf am selben Krankheitsbild beteiligte Mechanismen haben. Zu den durch die erfindungsgemäßen Verbindungen behandelbaren funktionellen Störungen zählen insbesondere die als sogenanntes "irritable bowel syndrome" (= IBS) oder Reizdarmsyndrom bekannten Störungen der unteren Darmwege. Wesentliche Symptome von IBS sind Unterbauchschmerzen, die auf einer Übersensibilität des visceralen afferenten Nervensystems zu beruhen scheinen, und Anomalien des Stuhlganges, insbesondere anomal beschleunigte Passage des Stuhls im Colon. Die erhöhte viscerale Schmerzempfindlichkeit gegenüber mechanischen oder chemischen Reizen im intestinalen Trakt führt dazu, daß IBS-Patienten schon bei physiologischen verdauungsbedingten geringen Dehnungen des Colons, z. B. bereits bei geringer Gasbildung und leichten Blähungen, die von Gesunden kaum wahrgenommen werden, starke viscerale Schmerzen erleiden. Zu durch die erfindungsgemäßen Verbindungen günstig beeinflußbaren inflammatorisch bedingten Störungen im gastrointestinalen Trakt gehören die im allgemeinen unter dem Begriff IBD (= inflammatory bowel disease) zusammengefaßten entzündlichen Störungen im Dünndarm- und Dickdarmbereich, u. a. Colitis ulcerosa und Morbus Crohn. Das Wirkprofil der Substanzen zeichnet sich durch gute orale Bioverfügbarkeit mit einer günstigen Selektivität der Neurokinin-Rezeptorantagonistischen Wirkungen gegenüber unerwünschten Nebenwirkungen aus. So wurde in Dosisbereichen, die den NK-1-Rezeptor blockieren, in pharmakologischen Testversuchen keine cardiovasculäre calciumantagonistische Wirkung festgestellt.

Die angegebenen Beispielsnummern beziehen sich auf die nachstehend beschriebenen Herstellungsbeispiele.

### Beschreibung der pharmakologischen Testmethoden

### 1. Bestimmung des Bindungsvermögens der Testsubstanzen an NK-1-Rezeptoren in vitro.

Die Affinität der Testsubstanzen zu humanen NK-1-Rezeptoren wird in vitro gemessen. Bestimmt wird die Hemmung der Bindung des physiologischen Neurokinins (Substanz P) an Neurokinin-1-Rezeptoren.

Die Rezeptor-Bindungsstudien werden mit [³H]-Substanz P als Ligand durchgeführt. Für den Bindungsversuch werden verschiedene Proben einer Membranpräparation von CHO-Zellen (= Eizellen des chinesischen Hamsters, chinese hamster oocytes), die den humanen NK-1-Rezeptor exprimieren, mit einer Lösung des markierten Liganden inkubiert, wobei die Inkubationsansätze keine Testsubstanz oder Zusätze unterschiedlicher Konzentrationen an Testsubstanz enthalten. Anschließend wird in den Proben jeweils eine Trennung von gebundenem und freiem Liganden mit Hilfe einer Glasfiber-Filtration vorgenommen. Die im Filter verbleibende Fraktion wird mehrmals mit Pufferlösung gewaschen und anschließend wird die Radioaktivität der im Filter verbliebenen Fraktion mit einem Beta-Scintillationszähler gemessen. Es wird als IC₅₀ der jeweiligen Testsubstanz diejenige Konzentration bestimmt, welche eine halbmaximale Verdrängung des gebundenen Liganden bewirkt. Aus dieser wird die entsprechende Inhibitionskonstante (Kᵢ-Wert) der Testsubstanz berechnet. In diesem Testmodell zeigte die Substanz des Beispiels 1 einen Kᵢ-Wert von 2,1 nmol/l für die Affinität zu humanen NK-1-Rezeptoren.

### 2. Bestimmung des Bindungsvermögens der Testsubstanzen an NK-2-Rezeptoren in vitro

Die Affinität der Testsubstanzen zu humanen NK-2-Rezeptoren wird in vitro gemessen. Bestimmt wird die Hemmung der Bindung der Verbindung SR-48,968 an NK-2-Rezeptoren. SR-48,968 ist eine als spezifischer NK-2-Antagonist bekannte synthetisch hergestellte Verbindung.

Die Rezeptor-Bindungsstudien werden mit SR 48,968 als Ligand durchgeführt. Die Versuchsdurchführung entspricht der im pharmakologischen Test zur Bestimmung des Bindungsvermögens der Testsubstanzen an NK-1-Rezeptoren in vitro angegebenen Methode. Im Unterschied hierzu werden nunmehr aber verschiedene Proben einer Membranpräparation von CHO-Zellen verwendet, welche den humanen NK-2-Rezeptor exprimieren. In diesem Testmodell zeigten die in der folgenden Tabelle 1 aufgeführten Beispielsubstanzen die angegebenen Kᵢ-Werte für die Affinität zu humanen NK-2-Rezeptoren:

**Tabelle 1: Affinität der Testsubstanzen zu humanen NK-2-Rezeptoren**

| **Beispiel-Nr.** | **Kᵢ [µmol/l]** |
|---|---|
| 1 | 0,06 |
| 2 | 0,05 |
| 3 | 0,30 |

### 3. Bestimmung des funktionellen NK-1-Antagonismus der Testsubstanzen an isoliertem Gewebe vom Meerschweinchen in vitro

Die NK-1-Rezeptor-antagonisierende Wirkung der Testsubstanzen wurde an isolierten, in einer oxygenierten Nährlösung gehaltenen Ringpräparaten der Aorta von Pirbright-White Meerschweinchen in vitro gemessen. Bestimmt wird die Hemmung der nach Stimulierung mit dem NK-1-Agonisten Substanz P hervorgerufenen Tonusrelaxation der Aortapräparate durch die Testsubstanzen.

Zur Messung der Kontraktion der Gefäßmuskulatur werden die Präparate an einem Haken fixiert, durch einen Faden mit einem Kraftmessungsgerät verbunden und die Kontraktionen werden jeweils an einem Schreiber registriert. Die Aortapräparate werden mit Phenylephrin tonisiert. Anschließend werden die NK-1-Rezeptoren der Präparate vor und nach der Gabe der Prüfsubstanz mit 0,01 µmol Substanz P stimuliert, wodurch eine Relaxation des Tonus herbeigeführt wird. Die Relaxationen vor und nach Gabe der Prüfsubstanz werden in Prozent quantifiziert. Als Kenngröße wird die Konzentration der halbmaximalen Hemmung (= IC₅₀) errechnet, welche diejenige Konzentration angibt, bei der eine halbmaximale Hemmung der Tonusrelaxation eintritt.

In diesem Testmodell zeigten die in der folgenden Tabelle 2 aufgeführten Beispielsubstanzen die angegebenen IC₅₀-Werte für die halbmaximale Hemmung :

**Tabelle 2: Funktioneller NK-1-Antagonismus der Testsubstanzen an isoliertem Meerschweinchen-Gewebe**

| **Beispiel-Nr.** | **IC₅₀ [µmol/l]** |
|---|---|
| 1 | 0,001 |
| 2 | 0,0012 |
| 3 | 0,0015 |

### 4. Bestimmung der Substanz-P-antagonistischen Wirkung der Testsubstanzen in vivo.

Zum Nachweis der Substanz-P-antagonistischen Wirkung der Testsubstanzen wurde als Standardtestmodell für Substanz-P-induzierte pharmakologische Effekte die durch Substanz-P-Applikation hervorgerufene Hypotension in Meerschweinchen verwendet. Es wurde die Hemmwirkung der Testsubstanzen gegenüber durch Substanz P induzierter Blutdrucksenkung nach intravenöser (= i.v.) und intraduodenaler (= i.d.) Applikation der Testsubstanzen bestimmt.

Männlichen Meerschweinchen werden in Narkose (Ketamin 67 mg/ kg, Xylazine 13 mg/kg) jeweils ein Katheter in eine Arteria Carotis Communis und in eine Vena Jugularis implantiert. Der arterielle Katheter dient zur Blutdruckmessung. Die Messung erfolgt mit einem Statham 23d/B Druckaufnehmer. Über den venösen Zugang erfolgt die Gabe von Substanz P bzw. bei intravenöser Gabe auch die Applikation der Testsubstanz. Nach einer Äquilibrierungsphase von 20 Minuten werden 50 pmol/Tier Substanz P als Bolus i.v. appliziert. Danach erfolgt die Gabe der Testsubstanz. Bei der i.v. Untersuchung wird die Testsubstanz in Dosierungen von 0.1, 0.46 bzw. 1.0 µmol/kg einer Gruppe von jeweils 4 bis 6 Tieren intravenös appliziert. Die Kontrollgruppe erhält die entsprechende Menge einer physiologischen Kochsalzlösung. Eine, 15, 30, 45 und 60 Minuten nach der Substanzgabe werden jeweils 50 pmol Substanz P i.v. appliziert. Bei den Versuchen mit intraduodenaler Substanzgabe wird abweichend zu der obigen Beschreibung zusätzlich ein Katheter im Duodenum der Versuchstiere implantiert. Die Testsubstanzen werden jeweils 3 bis 6 Tieren in Dosierungen von 0.046, 0.1, 0.46, 1.0, 4.6 und 10.0 µmol/kg über diesen Katheter appliziert. Als Vehikel dient bei diesen Versuchen Tylose. Es werden der mittlere arterielle Blutdruck vor und ca. 1 Minute nach der ersten Substanz-P-Gabe (vor Applikation der Testsubstanz) gemessen und daraus die maximale Substanz-P-induzierte Blutdrucksenkung bestimmt. Nach 60 Minuten werden die mittleren arteriellen Blutdruckwerte der nur mit Substanz P behandelten Kontrolltiere und der mit Substanz P und Testsubstanz behandelten Tiere verglichen und aus der Differenz wird die durch die jeweilige Testsubstanzdosis bewirkte Hemmung der Substanz-P-induzierten Blutdrucksenkung in Prozent, bezogen auf die maximale Blutdrucksenkung berechnet. Als ED₅₀ wird diejenige Dosis bestimmt, bei welcher eine 50%ige Hemmung der Substanz P-induzierten Blutdrucksenkung auftritt.

In diesem Testmodell zeigte die Substanz des Beispiels 1 nach i.v.-Applikation eine ED₅₀ von 0,2 umol/kg und nach i.d.-Applikation eine ED₅₀ von 0,08 µmol/kg. Dieses Verhältnis von i.d.- zu i.v.-Wirksamkeit kann als Indiz dafür gewertet werden, daß die Substanz gut zur oralen Applikation geeignet ist und daß ihre Wirkung bevorzugt im gastrointestinalen Trakt einsetzt.

In dem gleichen Testmodell werden die Testsubstanzen auch auf auf calciumantagonistischen Eigenschaften beruhende blutdrucksenkende Wirkungen untersucht. Hierzu werden Kontrolltiergruppen nur die Testsubstanzdosen ohne Substanz-P-Applikation verabreicht. Die Substanz des Beispiels 1 zeigte in dem untersuchten Dosisbereich (i.v.-Dosen bis zu 1 µmol/kg und i.d.-Dosen bis zu 10 µmol/kg) keine signifikante Blutdrucksenkung. Dies ist ein Indiz dafür daß in diesem Dosisbereich keine calciumantagonistischen Nebenwirkungen auftraten. Die überraschend geringen calciumantagonistischen Nebenwirkungen der erfindungsgemäßen Verbindungen lassen sich auch durch in vitro-Standardtestmodelle, beispielsweise an isoliertem Aorta-Gewebe von Meerschweinchen, nachweisen.

Die Substanzen können in üblichen pharmazeutischen Zubereitungen verabreicht werden. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 0,1 bis 80 mg, insbesondere 1 bis 10 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe, wie z. B. Talkum, Milchzucker oder Stärke neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nachfolgend angeführten Beispiele sollen die Erfindung näher erläutern, ohne sie in ihrem Umfang zu beschränken.

### Beispiel 1:

### (2R)-1-[3,5-Bis(trifluormethyl)benzoyl]-2-(1H-indol-3-ylmethyl)-4-(2-[N-(2-methoxybenzyl)aminoethyl]aminocarbonyl}piperazin

A) 101,5 g tert.-Butyloxycarbonylglycin wurden unter Stickstoffatmosphäre in 800 ml Dichlormethan gelöst und mit 96,5 ml Triethylamin versetzt. Unter Eiskühlung ließ man 58 ml Chlorameisensäureethylester langsam zutropfen, rührte das entstandene Gemisch noch 2 Stunden lang bei Raumtemperatur und tropfte anschließend eine Lösung von 79,8 g 2-Methoxybenzylamin in 400 ml Dichlormethan zu. Man ließ über Nacht rühren, gab dann 1.400 ml einer 15%igen wäßrigen Weinsäurelösung zu und ließ erneut 30 Minuten lang rühren. Anschließend wurde die organische Phase abgetrennt, über Natriumsulfat getrocknet und unter vermindertem Druck eingedampft. Der verbleibende Rückstand wurde aus Diethylether/Dichlormethan kristallisiert und im Hochvakuum getrocknet. Man erhielt 88,9 g N-BOC-C-(2-Methoxy) benzylaminoglycin als weißes Pulver, Fp. = 97° - 97,7 °C.
B) 40,0 g des vorstehend erhaltenen Produktes wurden unter Stickstoffatmosphäre in 600 ml eines Gemisches aus Toluol und THF (1:1) gelöst und zu einer eisgekühlten Vorlage von 21,0 g LiAlH₄ in 500 ml THF zugetropft. Man ließ das Gemisch über Nacht bei Raumtemperatur rühren und tropfte dann der Reihe nach ein Gemisch von 20 ml Wasser und 150 ml THF unter Eiskühlung und anschließend bei Raumtemperatur zuerst 20 ml einer 15%igen wäßrigen Natronlauge, gefolgt von 60 ml Wasser zu. Die überstehende Lösung wurde vom entstandenen Niederschlag abgesaugt und das Filtrat bei vermindertem Druck eingedampft. Man nahm den Rückstand in 240 ml einer 7,5%igen wäßrigen Weinsäurelösung auf und extrahierte die wäßrige Phase mit Dichlormethan. Anschließend wurde die wäßrige Phase durch Zugabe von 200 ml einer 10%igen wäßrigen Natronlauge auf pH 10 gebracht und noch 3 mal mit Dichlormethan extrahiert. Die vereinigten Dichlormethan-Phasen wurden über Natriumsulfat getrocknet, unter vermindertem Druck eingedampft und im Hochvakuum getrocknet. Man erhielt 28,0 g öliges N-BOC-N'-(2-Methoxy) benzyl-1,2-diaminoethan, welches ohne Reinigung weiter umgesetzt wurde.
C) 5,0 g des vorstehend erhaltenen Produktes wurden unter Stickstoffatmosphäre in 50 ml THF gelöst. Hierzu wurden 20 ml einer 1N wäßrigen Natronlauge gegeben. Unter Eiskühlung ließ man zu dem entstandenen Reaktionsgemisch gleichzeitig insgesamt 3,05 g Chlorameisensäurebenzylester und eine IN wäßrige Natronlauge so zutropfen, daß der pH-Wert 10 nicht unterschritten wurde. Nach vollendeter Zugabe wurde über Nacht bei Raumtemperatur gerührt. Anschließend gab man 150 ml Methyl-tert.-butylether zu, trennte die wäßrige Phase ab und wusch die organische Phase der Reihe nach mit 2 mal 50 ml Wasser, einmal 50 ml einer 15%igen wäßrigen Weinsäurelösung und erneut 2 mal mit 50 ml Wasser. Die organische Phase wurde dann über Natriumsulfat getrocknet, unter vermindertem Druck eingedampft und im Hochvakuum getrocknet. Man erhielt 4,9 g N-BOC-N'-(2-Methoxy)benzyl-N'-Cbo-1,2-diaminoethan, welches ohne Reinigung weiter umgesetzt wurde.
D) 4,8 g des vorstehend erhaltenen Produktes wurden in 50 ml Dichlormethan gelöst. Hierzu gab man 4,4 g p-Toluolsulfonsäure und ließ das Reaktionsgemisch über Nacht rühren. Anschließend wurde eine Lösung von 7,5 g NaOH in 75 ml Wasser zugegeben. Die organische Phase wurde abgetrennt, einmal mit 75 ml Wasser gewaschen und über Natriumsulfat getrocknet. Das Lösungsmittel wurde unter vermindertem Druck abgedampft und das Produkt im Hochvakuum getrocknet. Man erhielt 3,5 g öliges N-(2-Methoxy)benzyl-N-Cbo-1,2-diaminoethan, welches ohne Reinigung weiter umgesetzt wurde.
E) 2,0 g (2R)-1-[3,5-Bis(trifluormethyl)benzoyl]-2-(1H-indol-3-ylmethyl)-piperazin wurden in 100 ml Dichlormethan gelöst. Hierzu wurden nacheinander 0,6 g Triphosgen, gelöst in 20 ml Dichlormethan, und 12,0 ml Diisopropylethylamin, gelöst in 20 ml Dichlormethan, zugegeben. Man rührte das entstandene Reaktionsgemisch 1 Stunde lang bei Raumtemperatur und tropfte anschließend 2,8 g der vorstehend erhaltenen Aminoverbindung, gelöst in 20 ml Dichlormethan, zu. Das Reaktionsgemisch wurde noch 18 Stunden lang gerührt und anschließend der Reihe nach mit 10%iger wäßriger Kaliumhydrogensulfatlösung, Wasser und erneut mit gesättigter Natriumhydrogencarbonatlösung gewaschen. Dann wurde die Dichlormethanphase über Natriumsulfat getrocknet und unter vermindertem Druck eingedampft. Chromatographie des Rückstandes an Kieselgel (Laufmittel: Dichlormethan/Methanol 3:1) lieferte 2,5 g öliges (2R)-1-[3,5-Bis(trifluormethyl)benzoyl]-2*-*(1H-indol-3-ylmethyl)-4-{2-[N-(2-methoxybenzyl)-N-Cbo-aminoethyl]aminocarbonyl}piperazin, welches ohne Reinigung weiter umgesetzt wurde.
F) 2,5 g des vorstehend erhaltenen Produktes wurden in 400 ml Ethanol gelöst und mit 0,5 g 10%igen Palladiumkatalysators auf Aktivkohle versetzt. Anschließend wurde bei einem Wasserstoffdruck von 4 bar 6 Stunden lang hydriert. Man filtrierte vom Katalysator ab und dampfte das Lösungsmittel unter vermindertem Druck ein. Chromatographie an Kieselgel (Laufmittel: Dichlormethan/Methanol 9:1) lieferte 1,0 g rohe Titelverbindung, welche durch Behandlung mit HCl-gesättigtem Diethylether in das Hydrochlorid überführt wurde, Fp. = 138° bis 140 °C.

Nach den vorstehend beschriebenen Methoden können auch die in der nachfolgenden Tabelle 3 aufgeführten Verbindungen der Formel I hergestellt werden:

**Tabelle 3: Weitere Verbindungen der Formel I**

| **Beispiel-Nr.** | **R¹** | **R²** | **R³** |
|---|---|---|---|
| 2 | CH₃ | H | OCH₃ |
| 3 | H | F | H |

### Beispiel I:

### (2R)-1-[3,5-Bis(trifluormethyl)benzoyl]-2-(1H-indol-3-ylmethyl)-4-{2-[N-(2-methoxybenzyl)aminoethyl]-aminocarbonyl}piperazin enthaltende Tabletten

Man stellte Tabletten in folgender Zusammensetzung pro Tablette her:

| | |
|---|---|
| (2R)-1-[3,5-Bis(trifluormethyl)-benzoyl]-2-(1H-indol-3-ylmethyl)-4-{2-[N-(2-methoxybenzyl)amino-ethyl]-aminocarbonyl}piperazin-Hydrochlorid | 20 mg |
| Maisstärke | 60 mg |
| Milchzucker | 135 mg |
| Gelatine (als 10%ige Lösung) | 6 mg |

Der Wirkstoff, die Maisstärke und der Milchzucker wurden mit der 10%igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

## Patentansprüche

1. Verbindungen der allgemeinen Formel I 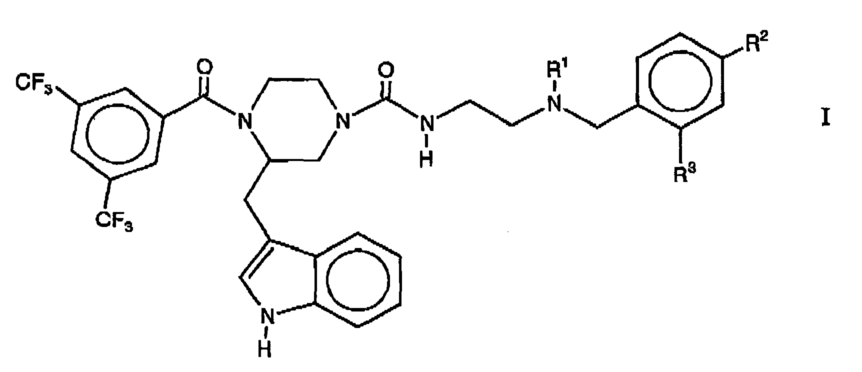 worin
R¹ Wasserstoff oder C1-C4 Alkyl bedeutet,
R² Wasserstoff oder Halogen bedeutet und
R³ Wasserstoff oder C1-C4 Alkoxy bedeutet
sowie deren physiologisch verträgliche Säureadditionssalze.

2. (2R)-1-[3,5-Bis(trifluormethyl)benzoyl]-2-(1H-indol-3-ylmethyl)-4-{2-[N-(2-methoxybenzyl)aminoethyl]-aminocarbonyl}piperazin und dessen physiologisch verträgliche Säureadditionssalze gemäß Anspruch 1.

3. Arzneimittel, enthaltend eine pharmakologisch wirksame Menge einer Verbindung gemäß Anspruch 1 und übliche pharmazeutische Hilfs- und/oder Trägerstoffe.

4. Verfahren zur Herstellung von Verbindungen der allgemeinen Formel I, 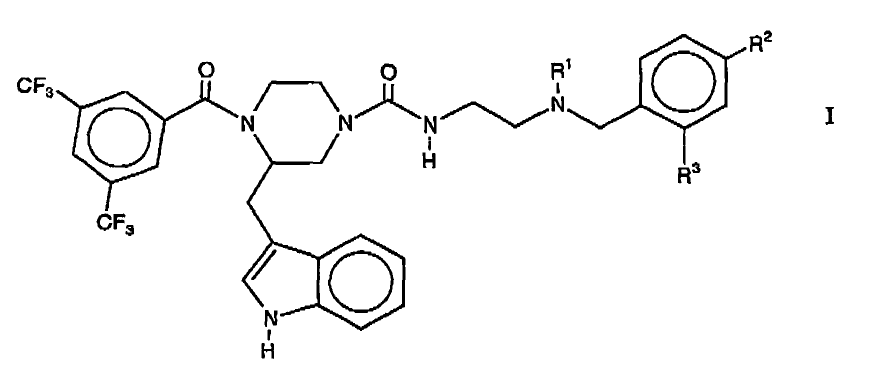 worin
R¹ Wasserstoff oder C1-C4 Alkyl bedeutet,
R² Wasserstoff oder Halogen bedeutet und
R³ Wasserstoff oder C1-C4 Alkoxy bedeutet
sowie deren physiologisch verträglichen Säureadditionssalzen, **dadurch gekennzeichnet, daß** man
a) die Verbindung der Formel II 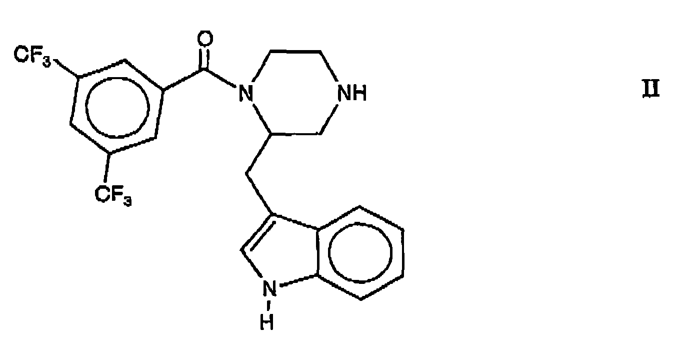 mit einer reaktiven Carbonylverbindung der allgemeinen Formel III, 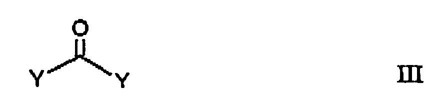 worin Y eine durch nucleophilen Angriff eines primären oder sekundären Amins verdrängbare Fluchtgruppe bedeutet, zu einer Carbamoyl-Verbindung der allgemeinen Formel IV, 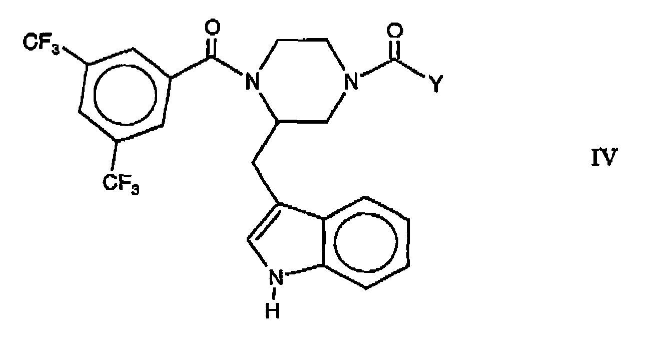 worin Y die obige Bedeutung besitzt, umsetzt, sofern durch Abspaltung der Fluchtgruppe Y aus einer Verbindung der Formel IV eine Säure entstehen kann, zu der erhaltenen Verbindung der Formel IV eine nicht-nucleophile organische Base zugibt, und die Verbindung der Formel IV anschließend mit einer Verbindung der allgemeinen Formel V, 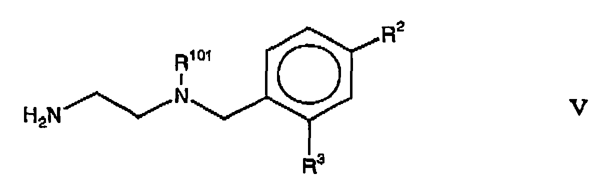 worin R¹⁰¹ C1-C4 Alkyl oder eine Aminoschutzgruppe bedeutet und R² und R³ obige Bedeutungen besitzen, umsetzt und eine allfällige Schutzgruppe R¹⁰¹ anschließend wieder abspaltet oder
b) die Verbindung der Formel II mit einer Verbindung der allgemeinen Formel VI, 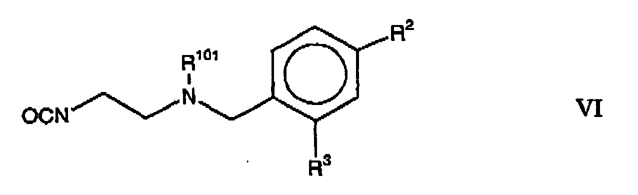 worin R¹⁰¹, R² und R³ obige Bedeutungen besitzen, umsetzt und eine allfällige Aminoschutzgruppe R¹⁰¹ nachfolgend wieder abspaltet
und gewünschtenfalls eine erhaltene Verbindung der Formel I, worin R¹ Wasserstoff bedeutet, zu einer Verbindung der Formel I, worin R¹ C1-C4 Alkyl bedeutet, alkyliert und eine erhaltene Verbindung der Formel I gewünschtenfalls in ihr Säureadditionssalz überführt oder ein Säureadditionssalz in eine freie Verbindung der Formel I überführt.

## Claims

1. Compounds of the general formula I 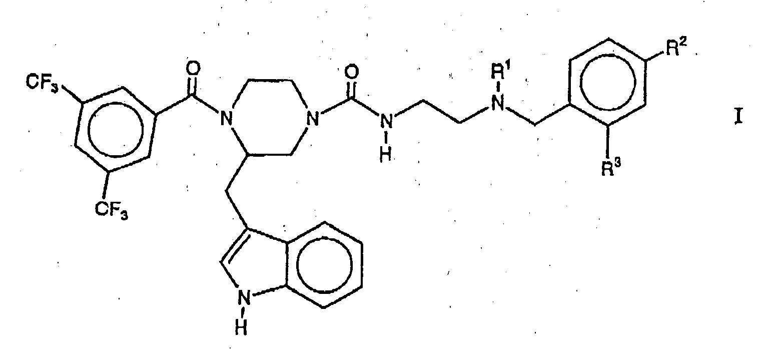 wherein
R¹ is hydrogen or C1-C4 alkyl,
R² is hydrogen or halogen and
R³ is hydrogen or C1-C4 alkoxy,
and their physiologically compatible acid addition salts.

2. (2R)-1-[3,5-bis(trifluoromethyl)benzoyl]-2-(1H-indol-3-ylmethyl)-4-{2-[N-(2-methoxybenzyl)aminoethyl]-aminocarbonyl}piperazine and the physiologically compatible acid addition salts thereof according to Claim 1.

3. Medicament, containing a pharmacologically effective quantity of a compound according to Claim 1 and conventional pharmaceutical auxiliaries and/or excipients.

4. A process for the preparation of compounds of the general formula I, 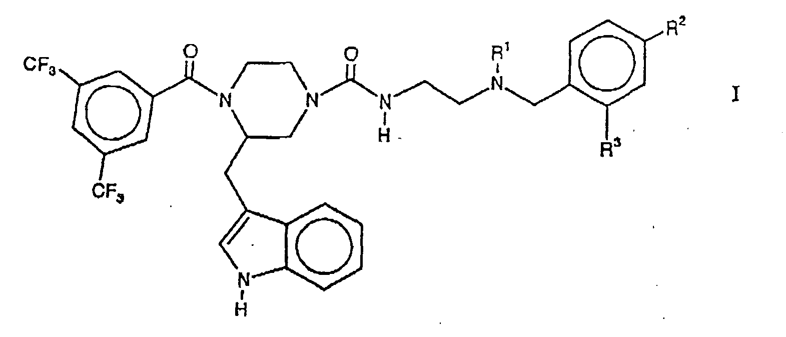 wherein
R¹ is hydrogen or C1-C4 alkyl,
R² is hydrogen or halogen and
R³ is hydrogen or C1-C4 alkoxy,
and their physiologically compatible acid addition salts, **characterised in that**
a) the compound of Formula II 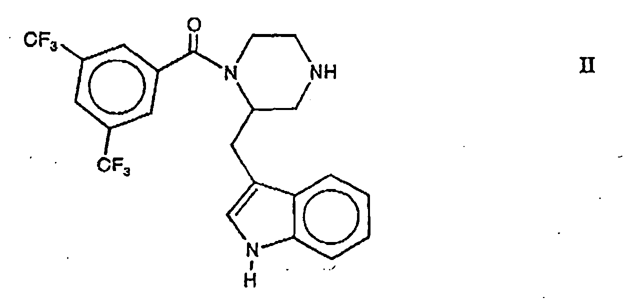 is reacted with a reactive carbonyl compound of the general formula III, 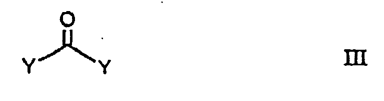 wherein Y represents a leaving group which can be displaced by nucleophilic attack of a primary or secondary amine, to form a carbamoyl compound of the general formula IV, 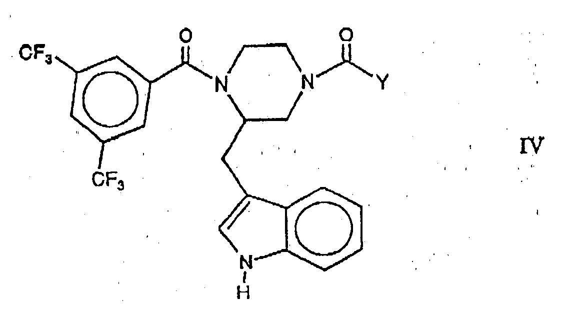 wherein Y has the above meaning, provided that by cleaving off the leaving group Y from a compound of Formula IV an acid can be produced, a non-nucleophilic organic base is added to the resulting compound of Formula IV, and the compound of Formula IV is then reacted with a compound of the general formula V, 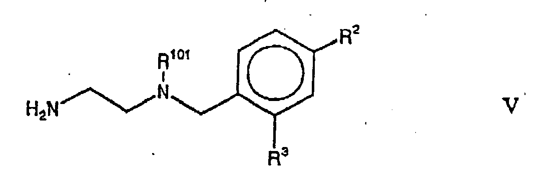 wherein R¹⁰¹ represents C1-C4 alkyl or an amino protective group and R² and R³ have the above meanings, and any protective group R¹⁰¹ is then cleaved off again,
or
b) the compound of Formula II is reacted with a compound of the general formula VI, 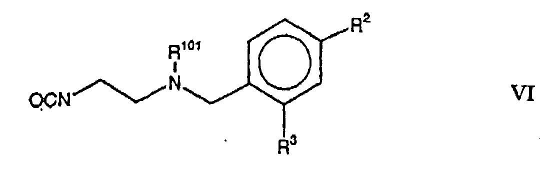 wherein R¹⁰¹, R² and R³ have the above meanings, and any amino protective group R¹⁰¹ is subsequently cleaved off again,
and if desired a resulting compound of Formula I wherein R¹ is hydrogen is alkylated to form a compound of Formula I wherein R¹ is C1-C4 alkyl and a resulting compound of Formula I if desired is converted into its acid addition salt or an acid addition salt is converted into a free compound of Formula I.

## Revendications

1. Composés de formule générale I 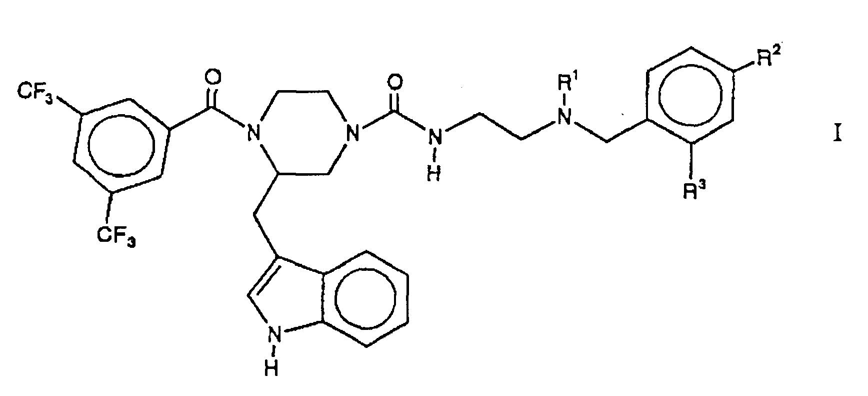 dans laquelle
R¹ représente un hydrogène ou un alkyle en C₁ à C₄,
R² représente un hydrogène ou un halogène et
R3 représente un hydrogène ou un alcoxy en C₁ à C₄,
ainsi que leurs sels d'addition d'acide physiologiquement acceptables.

2. (2R)-1-[3,5-bis(trifluorométhyl)benzoyl]-2-(1H-indol-3-ylméthyl)-4-{2-[N-(2-méthoxybenzyl)aminoéthyl]aminocarbonyl]-pipérazine et ses sels d'addition d'acides physiologiquement acceptables selon la revendication 1.

3. Médicament contenant une quantité pharmacologiquement efficace d'un composé selon la revendication 1 et des adjuvants et/ou supports pharmaceutiques habituels.

4. Procédé de préparation de composés de formule générale I 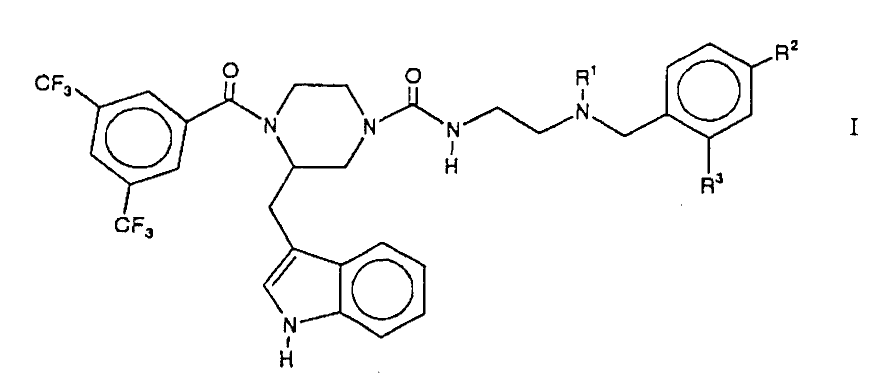 dans laquelle
R¹ représente un hydrogène ou un alkyle en C₁ à C₄,
R² représente un hydrogène ou un halogène et
R³ représente un hydrogène ou un alcoxy en C₁ à C₄,
ainsi que de leurs sels d'addition d'acides physiologiquement acceptables,
**caractérisé en ce que**
a) on fait réagir le composé de formule II, 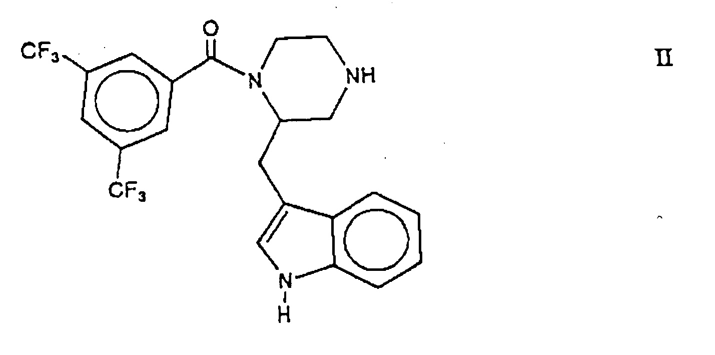 avec un composé carbonyle réactif de formule générale III, 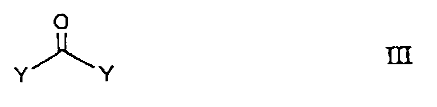 dans laquelle Y représente un groupe partant déplaçable par l'attaque nucléophile d'une amine primaire ou secondaire, pour donner un composé carbamoyle de formule générale IV, 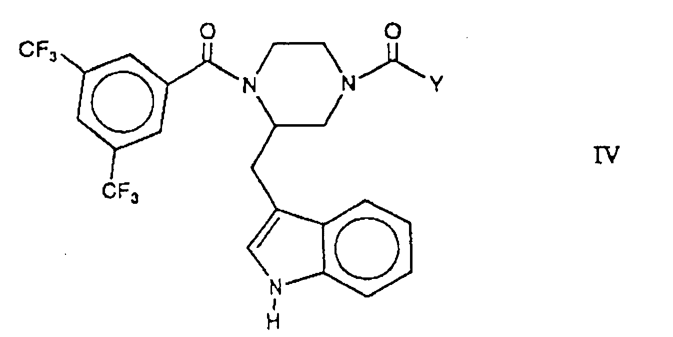 dans laquelle Y a la signification donnée ci-dessus, dans la mesure où par séparation du groupe partant Y à partir d'un composé de formule IV un acide peut être généré, pour donner le composé de formule générale IV obtenu, on ajoute une base organique non nucléophile au composé obtenu, puis on fait réagir le composé de formule IV avec un composé de formule générale V, 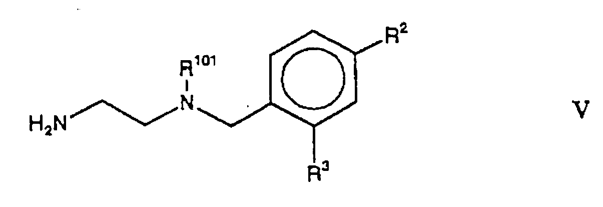 dans laquelle R¹⁰¹ représente un alkyle en C₁ à C₄ ou un groupe protecteur d'amino et R² et R³ ont les significations données ci-dessus, puis on sépare à nouveau un groupe protecteur R¹⁰¹ éventuel, ou
b) on fait réagir le composé de formule II avec un composé de formule générale VI, 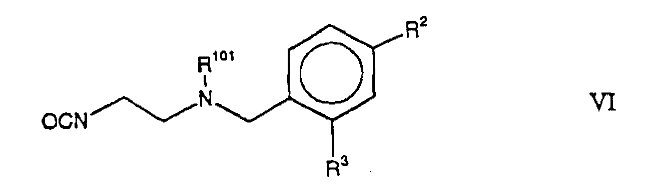 dans laquelle R¹⁰¹, R² et R³ ont les significations données ci-dessus, puis on sépare à nouveau un groupe protecteur d'amino R¹⁰¹ éventuel,
et si on le désire on alkyle un composé de formule I obtenu, où R¹ représente un hydrogène, pour donner un composé de formule I dans laquelle R¹ représente un alkyle en C₁ à C₄, et on transforme si on le désire un composé de formule I obtenu en son sel d'addition d'acide ou on transforme les sels d'addition d'acide en un composé libre de formule I.
